Europäisches Patentamt

European Patent Office

Office européen des brevets

(19)

(11) Publication number: 0 384 559
A1

## EUROPEAN PATENT APPLICATION

(12)

(21) Application number: 90300247.5

(22) Date of filing: 09.01.90

(51) Int. Cl.5: A61K 37/64

---

The title of the invention has been amended (Guidelines for Examination in the EPO, A-III, 7.3).

(30) Priority: 09.01.89 JP 2579/89

(43) Date of publication of application:
29.08.90 Bulletin 90/35

(84) Designated Contracting States:
AT BE CH DE DK ES FR GB GR IT LI LU NL SE

(71) Applicant: TAISHO PHARMACEUTICAL CO. LTD
24-1 Takata 3-chome Toshima-ku Tokyo 171(JP)

(72) Inventor: Katunuma, Nobuhiko
246-2, Myodocho-3-chome Tokushima-shi(JP)

(74) Representative: Lamb, John Baxter et al
MARKS & CLERK 57/60 Lincoln's Inn Fields London WC2A 3LS(GB)

(54) Use of trypstatin in the treatment of AIDS.

(57) Trypstatin significantly suppresses HIV infection without exhibiting cytotoxicity. The invention provides pharmaceutical compositions comprising trypstatin and the use of a trypstatin in the manufacture of pharmaceutical compositions for the treatment of AIDS.

EP 0 384 559 A1

## NEW PHARMACEUTICAL USE OF TRYPSTATIN

The present invention relates to a new pharmaceutical use of trypstatin and more particularly, to a new pharmaceutical use of trypstatin for the treatment of acquired immuno deficiency syndrome (AIDS).

Acquired immuno deficiency syndrome (AIDS) is a serious immunodeficiency induced by human immunodeficiency virus (HIV) and is now spreading worldwide. It has been desired to develop a drug for treating AIDS.

HIV, which is a causative agent of AIDS, is one of retroviruses having RNA as a genome. In 1983 it was isolated from lymphocytes of patients with AIDS by Montagnier et al in the Pasteur Research Institute and then in 1986 human immunodeficiency virus type 2 (HIV-2) was isolated from patients with AIDS in Western Africa by the same group [Mireidlle et al., Nature, 326, 662 (1987)].

It was revealed that HIV particles bind to CD4 antigens on the surface of helper T cells which play the central role in immunoreaction and invade into the cells to kill helper T cells, and this causes the immunodeficiency [Dalgleis et al., Nature, 312, 763 (1984); Klatzmann et al., Nature, 312, 767 (1984)].

It has also been revealed that infection of HIV is initiated by the binding of gp120, an envelope glycoprotein of HIV, with CD4 molecules on the surface of helper T cell [Dalgleis et al., Nature, 312, 763 (1984); Klatzmann et al., Nature, 312, 767 (1984); McDougal et al., Science, 231, 382 (1986)]. Based on this infection mechanism, an approach has been made using antibodies to gp120 as a drug to inhibit HIV infection [Robey et al., Proc. Natl. Acad. Sci, USA, 83, 7023 (1986)].

It was reported that the epitope of HIV neutralizing monoclonal antibody was located between amino acids 308-331 of gp120 [Matsushita et al., J. Viol., 62, 2107 (1988)].

Furthermore, it was reported that among the monoclonal antibodies to various synthetic oligopeptides comprising of a part of amino acid sequence of gp120, only the monoclonal antibody to the synthetic oligopeptide corresponding to the amino acids 303 - 321 inhibited the HIV infection [Palker et al., Proc. Natl. Acad. Sci. USA, 85, 1932 (1988)]. It is known that the region corresponding to the amino acids 303 - 321 is not involved in the binding of gp120 to CD4 [Kowalski et al., Science, 237, 1351 (1987); Lasky et al., Cell, 50, 975 (1987)]. Therefore, it may be possible to speculate that even an antibody to the region which is not involved in the binding to CD4 could modify the tertiary structure of gp120 and inhibit the binding of gp120 to CD4. It was reported, however, that an antibody to synthetic oligopeptide corresponding to the amino acids 504 - 518 in gp120 could not inhibit the syncytium formation while this antibody could bind to gp120 itself [Palker et al., Proc. Natl. Acad. Sci. USA, 85, 1932 (1988)].

From these facts, the regions corresponding to the amino acids 504 - 518, 303 - 321, and 308 - 331 can be recognized by the antibodies, but only the antibodies to the latter two regions can inhibit the HIV infection. Therefore, the latter two regions must be closely involved in the binding of gp120 to CD4. This region is located in the variable region, in which difference of amino acid sequence is often found among clinically isolated HIV.

On the other hand, a new protease inhibitor, trypstain, was recently isolated from mast cells of rat and its primary structure was determined [Kido et al., J. Biol. Chem., 263, 18104 (1988); Kido et al., Cell Engineering, 7, 851 (1988); Kido et al., Metabolism, 25, extra volume, entitled "Highlight of Metabolic Disease", 187 (1988)].

It is suggested that trypstatin would be closely involved in the formation of allergic inflammation associated with IgE [Kido et al., Cell Engineering, 7, 851 (1988); Kido et al., Biochem. Int., 10, 863 (1985)]. It is also known that trypstatin inhibits conversion of prothrombin to α-thrombin and also inhibits the action of activated blood coagulant factor X [Kido et a;, Biochem. Biophys. Res. Commun., 132, 436 (1985)].

Since trypstatin has such physiological activities, it has been desired to utilize trypstatin as medicines.

The present inventor has paid attention to the variable region of gp120 which will be closely involved in the binding of gp120 to CD4 molecule on the surface of helper T cell, which is the initial step of HIV infection. Among the variable region, the inventor found a relatively well conserved sequence, Gly-Pro-Gly-Arg-Ala-Phe. If this sequence is closely involved in the binding with CD4, a substance having a sequence similar to the aforesaid one would be expected to inhibit the binding of gp120 to CD4. As such substances, the present inventor has paid attention to trypstatin and made investigations on the inhibiting activity of syncytium formation which is an evidence of HIV infection. Then, it was found that trypstatin is a potent inhibitor of syncytium formation and is extremely effective as agents for the treatment of AIDS. The present invention has thus been accomplished.

## SUMMARY OF THE INVENTION

EP 0 384 559 A1

An object of the present invention is to provide a new medical use of trypstatin as an agent for the treatment of AIDS.

In one aspect, the present invention is directed to a pharmaceutical composition for the treatment of AIDS comprising a trypstatin as an active ingredient and a pharmaceutically acceptable carrier.

In another aspect, the present invention is directed to a method for treating AIDS which comprises administering a therapeutically effective dose of trypstatin to a mammal.

In further aspect, the present invention is directed to use of a trypstatin for the preparation of a pharmaceutical composition for treating AIDS.

## DETAILED DESCRIPTION OF THE PREFERRED EMBODIMENTS

The inhibiting activity of trypstatin against HIV infection according to the present invention is highly specific. That is, trypstatin of the present invention has higher activity by about 1000 times than inter-$\alpha$-trypsin inhibitor which is also one of the trypsin inhibitors and has a similar amino acid sequence. However, trypstatin of the present invention does not exhibit cytotoxicity, while inter-$\alpha$-trypsin inhibitor exhibits cytotoxicity at such high dose.

Trypstatin can be purified from, for example, mast cells of rat peritoneum, liver, lung, tongue, etc. [Kido et al., Cell Engineering, 7, 851 (1988); Kido et al, Metabolism, 25, extra volume, entitled "Highlight of Metabolic Disease", 187 (1988)]. For example, trypstatin can be purified from the homogenate of mast cells of rat peritoneum with appropriate combination of column chromatography, such as trypsinogen-sepharose 4B, Sephadex G-75 column, high performance liquid chromatography, and the like [Kido et al., J. Biol. Chem., 263, 18104 (1988)]. Human mast cell-derived trypstatin can also be used. Human trypstatin can be purified by the similar method. Alternatively, trypstatin can be chemically synthesized based on its known amino acid sequence [Kido et al., J. Biol. Chem., 263, 18104 (1988)] or, it can also be obtained with recombinant DNA technology by synthesizing the DNA sequence corresponding to the amino acid sequence.

It has been revealed by the present inventor that trypstatin can inhibit HIV infection. That is, when two human acute lymphoblastic leukemia cells, MOLT-4 (ATCC CRL-1582) and CEM/LAV cells which is obtained by persistent infection of LAV-1, one of HIV [Barre-Sinoussi et al., Science, 220, 868 (1983)], to CCRF-CEM cells (ATCC CCL-119), are co-cultured, syncytia are formed. If trypstatin is added to this system, the formation of syncytia is inhibited. Therefore, trypstatin can inhibit HIV infection.

Among trypsin inhibitors, trypstatin used in the present invention is a particularly potent inhibitor of HIV infection. On the other hand, trypstatin has no cytotoxicity. Therefore, trypstatin is extremely useful as drugs for the treatment of AIDS.

Trypstatin may be administered parenterally, e.g., intravenously, intraarterially, intramuscularly, pernasally, subcutaneously, intrarectally, etc., or orally.

Parenteral preparations such as intravenous, intraarterial, intramuscular and subcutaneous preparations include injections, infusions, etc. These pharmaceutical preparations may be prepared in a conventional manner. The injections, infusions, etc. may also be prepared by freeze-dried trypstatin alone or together with carriers such as mannitol, lactose, albumin, etc. and charging the freeze-dried product into a vial, etc. Trypstatin may also be formulated into liposomal preparations composed of a thin membrane of phospholipids such as lecithin, lysolecithin, sphingomyelin, etc. Trypstatin may also be formulated into implanted type preparations which may be inserted subcutaneously and maintained.

As the nasal preparations, there are powders comprising trypstatin together with carriers such as lower alkyl cellulose, e.g. hydroxypropyl cellulose, etc., polyacrylates, e.g., sodium polyacrylate, etc., crystalline cellulose, etc.

The intrarectal preparations include suppositories. The suppositories may be formulated by extensively dispersing trypstatin in a conventional base such as cacao butter, etc. in the presence of one or more surface active agents such as fatty acid esters, polyoxyethylene derivatives, pyridoxine fatty acid esters, sucrose fatty acid esters, phospholipids, etc. thereby to improve an absorption efficiency; and the like.

As the oral preparations, there may be exemplified the aforesaid liposomal preparations and the like.

The preparations described above may all be prepared in a conventional manner. If necessary and desired, these preparations may further contain therein preservatives, color agents, stabilizers, etc. which are conventionally used in the art.

A dose of trypstatin may vary depending upon age, body weight or conditions of patients, etc. but may be generally in the range from 0.1 to 500 mg/kg-body weight/day, preferably 10 to 100 mg/kg-body weight/day.

3

Trypstatin significantly inhibits HIV infection but shows no cytotoxicity. Therefore the pharmaceutical composition of the present invention comprising trypstatin as an active ingredient is highly useful for the treatment of AIDS.

The present invention will be described in more detail by referring to test examples.

Test Examples

1. Purification of trypstatin and human inter-α-trypsin inhibitor

Trypstatin was purified from rat tongue according to the method of Kido et al. [Kido et al., J. Biol. Chem., 263, 18104 (1988)]. The purified trypstatin had physical properties similar to trypstatin from rat peritoneum.

Human inter-α-trypsin inhibitor was purified from human sera according to the method of Wachter et al. [Wachter et al., Hoppe-Seyler's Z. Physiol. Chem., 362, 1351 (1981)].

2. Cells used

Human acute lymphoblastic leukemia cells, i.e., MOLT-4 cells (ATCC CRL-1582), and CEM/LAV cells obtained by persistent infection of CCRF-CEM cells (ATCC CCL-119) with LAV-1, which is one of HIV [Barre-Sinoussi et al., Science, 220, 868 (1983)], were used.

3. Incubation of cells

The cells were cultured in RPMI 1640 medium supplemented with 10% FCS under conditions of 37°C, 5% $CO_2$ and relative humidity of 95%.

4. Test on inhibition of syncytium formation

(1) Method

Test on inhibition of syncytium formation was performed in a manner similar to the method of Lifson et al. [Lifson et al., J. Exp. Med., 164, 2101 (1986)]. Briefly, a part of the culture of MOLT-4 cells was centrifuged at 2000 rpm for 5 minutes to collect the cells. After removing the supernatant, the cells were suspended in ASF 101 medium (manufactured by Ajinomoto Co., Ltd.) to prepare the cell suspension of $5 \times 10^5$ cells/ml. To this suspension, trypstatin or human inter-α-trypsin inhibitor at various concentrations was added. This suspension was dispensed into a 96-well microtiter plate ($1 \times 10^5$ cells/well) and allowed to stand at 37°C for 30 minutes. Two microliters of CEM/LAV-1 cells ($1 \times 10^7$/ml) was added to each well and then extensively mixed. After culturing at 37°C for 12 hours in the presence of 5% $CO_2$, syncytia formation was examined under an inverted microscope (magnification x 200). More than 60% inhibition of syncytia formation was evaluated as + +; more than 30 to 60% inhibition was evaluated as +; 30% or less inhibition was evaluated as ±; and when no inhibition was observed, it was evaluated as -. Thus, the inhibiting activity of syncytia formation was evaluated.

| (2) Results | | | | | |
| --- | --- | --- | --- | --- | --- |
| Concentration of Trypstatin (μM) | 0 | 0.3 | 1 | 4 | 10 |
| Inhibition of Syncytium Formation | - | + | + + | + + | + + |

As stated above, trypstatin significantly inhibited the syncytium formation depending on its concentration. 1 mM of human inter-α-trypsin inhibotor was necessary to exhibit 60% or more inhibition and cytotoxicity was observed at such a high concentration.

[Example 1]

Injections:

Freeze-dried trypstatin (600 mg) was dissolved in 6 ml of physiological saline for injection. After aseptically filtering through a filter (more diameter of 0.22 $\mu$m, manufactured by Millipore Co., Ltd.), 2 ml of the solution was charged in an ampoule and the space in the ampoule was substituted with nitrogen gas. The ampoule was sealed to give injections.

[Example 2]

Liposomal Preparations:

Yolk lecithin (110 $\mu$mol) and 50 $\mu$mol of cholesterol were dissolved in 10 ml of chloroform and the solution was charged in a flask of 100 ml. After extensively mixing, the solvent was evaporated on a hot bath of 40°C to form a thin layer on the inner wall of the flask. After drying under vacuum for 3 hours, trypstatin (30 $\mu$mol) dissolved in 10 ml of physiological saline was added. The mixture was vortexed and sonicated. After filtration through a membrane filter (0.22 $\mu$m, manufactured by Japan Millipore Industry Co., Ltd.), 10 ml of the solution was charged in an ampoule and the space in the ampoule was substituted with nitrogen gas. The ampoule was sealed to give liposomal preparations for injection.

[Example 3]

Suppositories:

To 17 g of cacao butter was added 1.0 g of sodium glycolate, and the mixture was extensively mixed in a mortar. Trypstatin (2 g) was taken into a mortar and the base prepared by mixing was gradually added thereto. The mixture was mixed to give a homogeneous suppository composition. The composition was charged in a container in an amount of 2 g to give suppositories.

[Example 4]

Powders for nasal administration:

Freeze-dried trypstatin (100 mg) and hydroxypropyl cellulose were mixed extensively in a mortar to give a uniform powdery composition. The composition was charged in a capsule to give trypstatin preparations for nasal administration.

## Claims

1. Use of a trypstatin for the preparation of a pharmaceutical composition for treating AIDS.

2. Use according to claim 1, wherein said trypstatin is a trypstatin derived from mast cells of human or rat origin.

3. Use according to claim 2, wherein said trypstatin is a trypstatin isolated from mast cells of the peritoneum, liver, lung or tongue of human or rat.

4. Use according to claim 2, wherein said composition is in a form sutitable for injection.

5. A pharmaceutical composition for the treatment of AIDS comprising a trypstatin as an active ingredient and a pharmaceutically acceptable carrier.

6. A pharmaceutical composition according to claim 5, wherein said trypstatin is as defined in claim 2 or claim 3.

7. A pharmaceutical composition according to claim 5 in a form suitable for injection.

8. A method for treating AIDS which comprises administering a therapeutically effective dose of a trypstatin to a mammal.

9. A method for treating AIDS according to claim 8, wherein said mammal is a human being.

10. A method for treating AIDS according to claim 9, wherein said trypstatin is as defined in claim 2 or claim 3.

11. A method fro treating AIDS according to claim 8, wherein said trypstatin is administered in an amount of from 0.1 to 400 mg/kg-body weight/day.

12. A method for treating AIDS according to claim 8, wherein said trypstatin is administered by injection.

European Patent Office

**PARTIAL EUROPEAN SEARCH REPORT**
which under Rule 45 of the European Patent Convention
shall be considered, for the purposes of subsequent
proceedings, as the European search report

Application number

EP 90 30 0247

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | PROC. NATL. ACAD. SCI. USA, vol. 85, June 1988, pages 4478-4482, Washington, US; J. GOUDSMIT et al.: "Human immuno-deficiency virus type 1 neutralization epitope with conserved architecture elicits early type-specific antibodies in experimentally infected chimpanzees" <br><br> * Page 4478, abstract * <br><br> -- | 1-7 | A 61 K 37/64 |
| D,A | THE JOURNAL OF BIOLOGICAL CHEMISTRY, vol. 263, no. 34, December 5, 1988, pages 18104-18107, The American Society for Biochemistry and Molecular Biology, Inc., Baltimore, MD, US; H. KIDO et al.: "Kunitz-type protease inhibitor found in rat mast cells" <br><br> * Page 18104, abstract; page 18105, figure 1 * <br> -- ./. | 1-7 | TECHNICAL FIELDS SEARCHED (Int. Cl.4) <br><br> A 61 K |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-7

Claims searched incompletely:

Claims not searched: 8-12

Reason for the limitation of the search:

Method for treatment of the human or animal body by surgery or therapy (see Art. 52(4) of the European Patent Convention).

| Place of search | Date of completion of the search | Examiner |
|---|---|---|
| THE HAGUE | 23-04-1990 | RIJCKEBOSCH |

| DOCUMENTS CONSIDERED TO BE RELEVANT | | | CLASSIFICATION OF THE APPLICATION (Int Cl 4) |
|---|---|---|---|
| Category | Citation of document with indication, where appropriate, of relevant passages | Relevant to claim | |
| A | PROC. NATL. ACAD. SCI. USA, vol. 85, September 1988, pages 6612-6616, Washington, US; S. SEELMEIER et al.: "Human immuno-deficiency virus has an aspartic-type protease that can be inhibited by pepstatin A" | | |
| | * Page 6612, abstract * | 1-7 | |
| | -- | | |
| P,X | FEBS LETTERS, vol. 248, nos. 1,2, May 8, 1989, pages 48-52, Elsevier Science Publishers B.V. (Biomedical Division), Amsterdam, NL; T. HATTORI et al.: "Involvement of tryptase-related cellular protease(s) in human immunodefi-ciency virus type 1 infection" | | TECHNICAL FIELDS SEARCHED (Int Cl 4) |
| | * The whole article * | 1-7 | |
| | ---- | | |

EPO Form 1505.3   06.78